# EUROPEAN PATENT APPLICATION

(11) **EP 4 052 743 A1**
(43) Date of publication of application: **07.09.2022**
(21) Application number: 21159938.6
(22) Date of filing: 01.03.2021
(51) Int. Cl.: A61M 5/20, A61M 5/315, A61M 5/48

(54) **CODE INFORMATION FOR DRUG DELIVERY DEVICE**

(71) Applicant: Ypsomed AG, 3401 Burgdorf (CH)
(72) Inventor: Schrul, Christian, 3400 Burgdorf (CH); Bosshard, Simon Martin, 3006 Bern (CH); Brügger, Martin, 3065 Bolligen (CH); Scheurer, Simon, 3006 Bern (CH)
(74) Representative: Meier Obertüfer, Jürg

(57) **Abstract**

The present invention relates to a drug delivery device (1) for dispensing a liquid drug. The device (1) comprises a receptacle (2) adapted to contain the liquid drug and is provided with a machine-readable code (76) including drug information, a drive member (20) for dispensing the drug from the receptacle (2), a code reader (90) for reading that information, an input value and a controller. That information comprises a first selection parameter value associated with a first drug delivery instruction and a second selection parameter value associated with a second drug delivery instruction. The controller is configured to compare the input value with the first and the second selection parameter value and to select one of the associated first or second drug delivery instruction based on the comparison.

## Description

### FIELD OF THE INVENTION

The present invention relates to injection devices or medicament delivery devices for injecting, delivering, administering, infusing or dispensing substances and/or liquids such as insulin, hormone preparations or vaccines. It departs from a drug delivery device comprising a receptacle comprising a liquid drug and a code with drug information. The drug delivery device further comprises a code reader for reading that information.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Drug delivery device based therapies generally benefit from an electronic unit or control unit embedded or integrated in the delivery device, or being part of an auxiliary or supplemental electronic module or add-on device detachably attached to the delivery device. The electronic unit monitors a drug delivery process, in order to proactively prevent false handling of the device and/or to keep track of the doses already applied, and generates data related to an instantaneous condition and/or use of the delivery device. Suitable sensors of the electronic unit readily detect a status or signal from any kind of indicating component of the delivery device, including user interface elements and actuators. A wireless communication unit of the electronic unit is provided to wirelessly communicate, specifically upload, drug information to a nearby mobile device, a dedicated medical gateway or a cloud server. The drug information includes at least a time stamp and the expelled dose, indicative of a time of a medication event and of a quantity of delivered drug. The drug information may be transmitted instantaneously, or stored in a memory unit connected to the processing unit, for later upload or batch transfer.

The electronic unit may be able to read information from a cartridge or cartridge unit. In particular reusable devices that allow to replace a cartridge may comprise a sensor that may read drug related information from a cartridge tag or cartridge code. That read information may be processed and stored by the electronic unit in the delivery device or additionally uploaded by the communication unit.

For example, WO16033507A2 describes an automatic injector including a housing adapted to receive and support a syringe. The syringe comprises a tag in form of a near-field communication tag. That information in the tag includes an expiration date of the drug, a manufacturing identity and a drug ID, a serial number of the syringe, an amount or required dose of drug to be delivered, a required dose delivery rate, a temperature of drug at time of delivery and other operational parameters necessary for the accurate delivery of the drug medicament by the automatic injector. The authenticity of the data of the syringe may be determined based on the manufacturer ID that are included in that information received from the cartridge tag. A control unit of the automatic injector determines that there is a match in an external or internal database to the received manufacturer ID. Based on the validation of the ID the control unit may notify the user that the data received from the tag is authentic.

US2019143043A1 discloses an injector with a motorized needle insertion mechanism. The injector comprises a removable cassette having a container with a medicament. The cassette comprises a RFID chip on an exterior surface of a bottom wall of a cassette outer housing. The injector includes sensors to detect the chip. The injector can automatically select operating parameters for injecting the drug, such as injection speed, needle insertion speed, pre and post-injection wait time, needle insertion depth and temperature limits based on information provided by the RFID chip.

US2019009029A1 discloses an injection pen with an electric motor and a replaceable cartridge inside the pen. The cartridge comprises an NFC tag. The tag is indicative of an optimal speed of stopper movement, such as stopper movement in different phases of movement, during air-shot, and/or injection and of a suitable dwell time for the medicament. A processing unit of the auto injector is configured to determine the cartridge specifications based on the ID number by table lookup. A processing unit of the injection pen is configured to determine an unauthorized cartridge, such as a counterfeit cartridge, a used cartridge, a tampered cartridge, a cartridge containing a wrong dose or a cartridge containing a wrong medicament, based on a code signal from the cartridge tag.

EP2526986B1 discloses an electronic injection pen comprising a syringe inside a housing. A microprocessor of the pen compares information read from an identification code of the syringe with criteria stored in the memory of the pen. If read syringe information matches the stored criteria the injection processing is performed. If it is determined that it is a wrong syringe an error message is displayed on the display section. Additionally, the electronic dispensing mechanism is blocked to prevent a dispensing of the medication with the wrong syringe.

Delivery instructions and delivery commands provided in by the tag or code of these prior art approaches are fix and non-variable. Parameters mentioned in prior art relate to predefined condition, e.g. an ideal drug delivery environment or initial parameters which may not be appropriate for an instantaneous drug delivery situation or an instantaneous user condition.

### DESCRIPTION OF THE INVENTION

It is an objective of the present invention to enable an accurate and user-specific administration by means of a drug delivery device.

This objective is achieved by the drug delivery device and the method for determining a drug delivery instruction according to the independent claims. Preferred embodiments are evident from the dependent claims.

According to the invention a receptacle of the drug delivery device comprises or is provided with a machine-readable code with information. The drug delivery device comprises a code reader for reading that code information and a controller for subsequently processing of the read information in the delivery device. The controller of the drug delivery device is configured to select or to choose a non-void drug delivery instruction provided in that read code information based on a preceding comparison of a present, actual, up-to-date, real-time or instantaneous parameter value not comprised in that code information (but provided to the controller from a different source) to two or more predefined parameter values comprised in that code information. The selection parameters value preferably comprise a value or a value range. Non-limiting examples of a selection parameter value is an environmental parameter value (temperature, air pressure), a physiological body parameter value (body temperature, body weight, blood value) or an injection parameter value (size of a dose, number of injection, a current position of the drive member). The rules or criteria for the selection are preferably provided by the code information. Alternatively, the rules or criteria may be stored in the drug delivery device. Preferably, several parameters and/or criteria are evaluated in series or parallel, leading to a combination of delivery instructions.

An input value is provided to the controller. This input value may be, for example, a measured value, a user input or a value received via data transmission from an external sender. The controller is adapted to compare the input value with the selection parameter value. Comparing implies that the controller decides whether the input value corresponds to a specific selection parameter value or whether the input value lies within a selection parameter value range or if the input value fulfills a predefined criteria related to a specific selection parameter value. Alternatively, the controller may determine if the input value lies above or below a selection parameter value.

Based on the comparison the controller selects or choses the appropriate drug delivery instruction that is associated with or linked to the concerned selection parameter value. Preferably, the code comprises more than two selection parameters values, preferably more than five selection parameters values, wherein each selection parameter value is associated with a drug delivery instruction. Examples of a delivery instruction include a specific dose size, a feed rate (delivery rate), a holding time or an indicated injection or infusion site. Different input values incur distinct delivery instructions, quantitatively (small or large dose size, short or long holding time) of the same instruction type. Additionally or instead, an input value may also incur a delivery instructions of a different instruction type.

According to the invention the first selection parameter is associated with a first drug delivery instruction and a second selection parameter is associated with a second drug delivery instruction. That means if the input value corresponds to or is related somehow to a certain selection parameter the corresponding drug delivery instruction has to be considered. In mathematical terms, the delivery instruction is function of the input value. Examples of a delivery instruction are a device parameter such as a feed rate, a delivery rate or a delivery parameter such as a dose size or a holding time or a specific user instruction for the drug delivery such as an indication for a specific injection site to be used.

Contrary to prior art approaches the delivery parameters or instructions are not determined based on unalterably (non-variable) cartridge code information. Instead, the controller can select or choose from at least two drug delivery instructions that are not only based on the drug but also on instantaneous environmental, user and/or delivery device factors. As an example, the delivery device sets the delivery rate or feed rate based on the type of the drug, based on the current temperature (viscosity of the drug), based on an age of the user and based on the size of the dose. Such a sophisticated selection of drug delivery parameters or instructions allows an accurate administration and absorption of the liquid drug. A wide range of applications or therapies are possible and easily arranged for with one single delivery device type. Alternatively and not preferably an exhaustive set of all potential delivery instructions are stored in a local memory of the drug delivery device.

The user may manually adjust or amend the delivery instruction set by the controller. In particular, the user may input data or make adjustments taking into account actual environmental circumstances or physical conditions of the user.

The code of the receptacle is preferably readable by the code reader if the receptacle is attached to or inserted into a drive unit or a dispensing mechanism. For this purpose, the code reader is preferably located near or close to the code of the receptacle in an attached state or mounted state. After reading that information from the code the controller processes that read data from that information.

That read information from the code may be, for example, stored in a storage module of the delivery device, displayed to the user and/or wirelessly transmitted to an external receiver such as a cloud server, a HCP computer or healthcare facilities. The code and the code reader as described above may be part of an enhanced therapy concept including cloud services and remote user guidance. Namely, read information from the code may be transmitted from the drug delivery device to a cloud server. The user, device and/or drug information may be verified and compared with an external user-specific therapy plan or by a health care practitioner (HCP). Depending on the verification the user may be informed via display on the delivery device and/or via user device. Such connectivity features are described in the patent application of EP20216386.1 filed on 22.12.2020. Its disclosure is incorporated hereby by reference.

In a preferred embodiment the drug delivery device does not have to be connected to an external device. Information provided by the code of the receptacle and the input value (preferably entered by the user or measured by a sensor of the delivery device) are sufficient to determine an optimal delivery parameter for the drug delivery operations. In other words, information contained in the code of the receptacle allows the controller to determine and set the optimal parameters for the subsequent delivery of the drug.

The term "selection parameter value" is not limited to a single value or character. The selection parameter value may comprise several values, characters, value range or a number range. The selection parameter value may be a value in a value range, for example an end value, a limit value or a threshold.

As mentioned above the input value may be, for example, a measured value, a value stored in a storage module of the delivery device, an input value indicated by the user via user interface or a value received via data transmission. The input value may be, for example, an environmental value or administration parameter measured by a sensor of the delivery device. An example for such a value may be a current ambient temperature or drug temperature. Further the input value can be drug information monitored or registered by the controller during previous drug delivery events such as the number of dispensing events with a specific cartridge or drug, a cumulative amount of drug already dispensed from the reservoir or the position of a drive member (e. g. plunger rod, drive sleeve) of the delivery device relative to a device housing and determined by a position sensor of the delivery device. Furthermore, the input value may be a physiological body parameter such as a body weight, a blood value or any other biometric parameter or a therapy parameter such as, for example, a medication type, maximum or minimum dose values, an administration time table.

The receptacle may be a housing or holder comprising a cartridge, a container or a reservoir with the drug. Alternatively, the receptacle may be a cartridge or container including the liquid drug. In the latter case the machine-readable code is directly accommodated by or mounted to the cartridge or container.

In a preferred embodiment the receptacle comprises a cartridge or reservoir encasing the liquid drug and a housing or holder enclosing the cartridge or reservoir. In this embodiment the machine-readable code is preferably arranged in or onto the housing or holder or sandwiched in-between the cartridge and the housing. The cartridge or reservoir is preferably made of transparent plastic or glass such that the liquid drug is visible from outside.

The machine-readable code may be implemented in form of optical code or a code readable based on electromagnetic fields. Examples for an optical code are an Object Identifier code (OID), QR codes, bar codes, color codes and optical patterns. Examples for codes based on electromagnetic fields are a radio-frequency identification (RFID) tags or near-field communication (NFC) tags.

Examples for a drug delivery device may be a disposable injection pen comprising a cartridge with the liquid drug, a semi-disposable injection pen with a reusable drive unit and a replaceable receptacle including a cartridge, a reusable injection pen with replaceable cartridge, an autoinjector comprising a syringe (in particular a semi-disposable autoinjector with a reusable drive unit and a replaceable receptacle including a syringe) or a patch injector.

The drive member may be, for example, a plunger rod, a drive sleeve, a clutch sleeve or any member moving relative to a delivery device housing during a dose dispensing operation of the delivery device.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

Preferably, the first and second selection parameter value are temperature values or temperature ranges and the input value is an instantaneously measured temperature value. The temperature is preferably a measured ambient temperature or a measured temperature representing the temperature of the drug. The drug temperature can be, for example, measured on a temperature analogue (e.g. a member of the delivery device that has the same or nearly the same temperature than the drug) or on an outside of a cartridge or reservoir encasing the liquid drug with a temperature sensor of the delivery device. The ambient temperature may be measured with a temperature sensor of the delivery device or alternatively the current measured temperature value may be wirelessly received via a communication module from an external sender.

The drug delivery instruction is preferably a feed rate (delivery rate) for the drive member. That means the controller is adapted to select or to choose a feed rate of code information depending on the measured temperature value as a certain feed rate is associated with a certain temperature. The current optimal feed rate depending on both the drug and the current temperature can thus be determined for the dispensing. That is advantageous as the viscosity and thus flow properties of the liquid drug are heavily depending on the temperature.

In another embodiment the first and second selection parameter value are dose values or dose value ranges and the input value is a dose value to be dispensed. The dose value as input value may be a predefined value stored in a storage unit. Alternatively, the input value may be defined by a therapy plan stored in a storage module in the delivery device or received via communication module in the delivery device form an external sender, for example, from a cloud server or a user device. In the latter case the controller may read drug information from the tag and determines if the drug (drug ID, type of drug, expiry date) matches the data of a therapy plan. Consequently, the controller receives the dose value according the therapy plan stored on a cloud server or in a storage of the delivery device.

The controller is adapted to select or to choose an optimal feed rate given in code information depending on the dose value (stored or entered by the user) as a certain feed rate is associated with a certain dose value. Thus, the chosen accurate delivery rate depends on both the size of the dose and the drug.

In yet another embodiment the first and second selection parameter value are position values or position value ranges of a position of the drive member relative to a delivery device housing and the input value is a measured current position of the drive member relative to the device housing. The drive member may be, for example, a plunger rod, a drive sleeve, a clutch sleeve or any member moving during a dose dispensing operation of the delivery device. In this embodiment the delivery device comprises a sensor configured to detect a movement and/or position of the drive member relative to the device housing.

The controller is configured to determine the optimal feed rate given in that code information depending on the current position of the drive member as a certain feed rate is associated with a certain drive member position. That means the feed rates may vary during the dispensing operation. For example, the feed rate may be low at the beginning when the drive member starts to move compared to the rest of the dispensing movement or/and to the drive member may be moved slower when it reaches an end of the dispensing operation. Hence, the controller is configured to move the drive member on both depending on the current position of the drive member and depending on the drug.

In another embodiment the first and second selection parameter value are temperature values or temperature ranges, the input value is an instantaneous measured temperature value and the drug delivery instruction comprises a holding time to be indicated to the user.

The holding time or dwell time is the time the user has to hold the delivery device onto the injection site or infusion site before removing the delivery device. If the delivery device is removed before the holding time has lapsed the liquid drug may not completely be administered. The holding time can be indicated to the user via user interface on the delivery device. Additionally or instead, the controller transmits the value of the holding time to a user device where the holding time can be displayed to the user.

A liquid drug with a higher viscosity (lower temperature) may require a longer holding time compared to a liquid drug with a lower viscosity (higher temperature). Contrary to prior art approaches the holding time read from that tag information is not a constant value. Instead, the controller is adapted to select the optimal holding time given in that code information depending on the (current) measured temperature as a certain holding time is associated with a certain temperature. That allows to indicate the current optimal holding time depending on the temperature and thus the viscosity of the drug.

Preferably, the first and second selection parameter value are dose values or dose value ranges, the input value is a dose value and the drug delivery instruction comprises a holding time to be indicated to the user. In this embodiment the holding time is depending on the size of the dose to be dispensed. Hence, the controller is adapted to determine or to choose the optimal holding time depending on the dose value as a certain holding time is associated with a certain dose size. That is advantageous as a larger dose size may require a longer holding time to ensure that the dispensed dose can be fully absorbed by the user.

In yet another embodiment the first and second selection parameter value are physiological body parameter or parameter ranges and the input value is an entered or measured physiological body parameter of the user. Non-limiting examples of body parameters are the body weight, the body height, a body temperature, a blood value (e.g. blood sugar value, blood pressure) or any value representing a physical condition of the user. The injection instruction preferably comprise a dose size or dose amount to be dispensed.

The input value in form of a body parameter may be entered by the user via a user interface on the drug delivery device or alternatively the input value may come from an external sender, for example, from a cloud server, a smart device, wearables (smart watch, smart ring, smart bracelet) or a HCP. In this case the data may be received via a communication module in the delivery device.

The controller is adapted to select a dose given in that code information depending on the body parameter of the user as a certain dose size is associated with a certain body parameter. Hence, the controller dispenses a dose that depends on a physiological body parameter and on the drug. The dose to be dispensed may additionally be determined based on a therapy plan or based on a user input.

In an alternative embodiment the input value may be information from a user specific therapy plan which may comprise information about the dose size, the drug and the time of an administration as described above. Accordingly, the controller may choose a dose indicated in code information depending on the user specific therapy plan.

Additionally or instead, the controller may indicate to the user via a display on the delivery device or via a user device an appropriate injection or infusion site. The injection or infusion site may depend on both the drug and the actual body parameter of the user. Namely, a certain body parameter (e. g. blood value, body weight) may require a specific dose size and the dose size may require a specific injection or infusion site.

Furthermore, the first and second selection parameter value may be a user-specific therapy parameter value or parameter range and the input value may be an entered or measured physiological body parameter value of the user. The delivery instruction is preferably a dose size (dose amount) to be delivered by the delivery device. That means the code of the receptacle has been written with user-specific therapy data. When the user inserts or attaches the receptacle user-specific information is read by the controller. The user inputs his actual body parameter value or the controller receives the actual body parameter value via a communication module. Subsequently, the controller determines a specific therapy parameter value according to the user-specific therapy plan given in that code information and depending on the body parameter value of the user as a certain therapy parameter value is associated with a certain body parameter.

Examples of therapy parameter values are a dose size, a type of a dose (variable, fix) a maximum settable dose, a lock time (e. g. a minimum time period to wait unit the next administration), specific administration times and specific injection gradients.

As an example, the controller may read therapy information including the maximum settable dose and the administration time from the code. The user enters an actual blood sugar value and the controller adjusts the maximum settable dose and the administration time based on the actual blood value. Accordingly the user can enter a dose size and the controller sets the dispensing parameters value (e.g. dose, feed rate, holding time) which are still within the user-specific therapy plan parameters.

In another embodiment the first and second selection parameter values are numbers or number ranges and the input value is a counted number of drug delivery events with the drug delivery device. In this embodiment the controller preferably counts the number of delivery event with the same drug originating from the same receptacle.

The drug delivery instruction is preferably the dose size or dose amount. Thus, the controller is configured to select or choose the optimal dose size given in that code information depending on the already dispensed doses. That means a first dose may be smaller or larger than a second or further dose with the same drug.

In a preferred embodiment the first and second selection parameter values are values or value ranges representing a spatial orientation of the delivery device and the input value is a measured spatial orientation of the delivery device. The spatial orientation of the delivery device indicates how (what direction) the user holds the device. If the user holds the device with the needle end upwards it is likely that the user intends to carry out a priming. If the user holds the device with the needle end upside down it is likely that the user intends to administer a dose with the device. The delivery device preferably comprises a sensor configured to determine the spatial orientation of the device such as, for example, an inertial measurement unit.

The drug delivery instruction preferably comprises a dose size or dose amount to be dispensed and the controller is adapted to determine the size of the dose depending on the measured spatial orientation of the device as a certain dose size is associated with a certain spatial orientation. This is advantageous as, for example, the controller may set only a very small dose for a priming operation if the device is held needle end upwards. A regular dose may be set if the user holds the device needle end upside down.

Preferably, the drive member is prevented from a dispensing movement if the input value exceeds a predefined threshold of the parameter type provided in that code information. This is a safety feature as the dispensing of a dose can only be carried out if a predefined condition is met. The controller blocks or stops the drive member if, for example, a measured temperature is too high or too low, if that read code information does not match the data of the therapy plan, if stored data in the delivery device or if a physiological body parameter of the user (e.g. user body weight, blood sugar value) is not appropriate or does not match for a specific drug.

Furthermore, the threshold may be a predefined time period after an injection or infusion, wherein during this time period the controller prevents a dispensing operation. Thus the required time to wait after an administration before the next administration can be carried out can be kept.

Alternatively, the threshold may be a predefined daytime during that time the controller prevents a dispensing operation or a specific dose size (for example a large dose is not permitted at night).

In a further embodiment the threshold may depend on an input value such as a predefined therapy plan, a physiological body parameter or a user input. That means, for example, as long as a physiological body parameter of the user (e.g. a blood sugar value) is not in accordance with a predefined value (e. g. a predefined blood sugar value) stored on a cloud server or in a storage of the device the controller prevents a dispensing operation.

In a further embodiment the code may comprise instructions for use that are displayed to the user via user interface, e. g. via display on the delivery device and/or via user device. The instructions for use preferably relate to the drug itself, to the delivery of the drug and/or to the delivery device. As an example, the instruction for use may comprise instructions how to prime a specific drug or how to prepare the injection site or infusion site or indicates an appropriate injection site for a specific drug. If the drug comprises more than one component the instruction for use may comprise instructions how to prepare the drug for the administration, for example, how to mix the components of the drug. The instructions for use may comprise additional information such as information about the drug, side effects of the drug or any safety instructions.

Additionally, to the above described comparing of the input value with the first and the second selection parameter value the controller is preferably configured to compare received data from an external sender with the first and the second selection parameter value and to choose the drug delivery instruction accordingly. That means the dispensing parameters are set by the controller at least based on that drug information read from the tag, on the input value and on the received data. The latter may be, for example, dose information, dose amount or conditions from a user-specific therapy plan. The external sender may be a cloud server, a user device, a computer of a HCP or health facility.

In a preferred embodiment the machine-readable code is a near field communication tag (NFC tag). Such a tag is readily available and can be implemented on low costs. Alternatively, the code may be an optical readable code such as OID code, a bar code, a color code, a grey code or the like.

Additionally to the above described code features the code preferably comprises additionally at least one control command for controlling the drug delivery device. Such control commands preferably control a dose and dispensing mechanism of the delivery device. In particular, the control commands may disable, enable or restrict a dose dispensing movement of the drive member depending on a predefined criteria or enable, disable or restrict a dose setting and/or dose correction movement of a dose setting member of the delivery device depending on a predefined criteria. Furthermore, the control commands may comprise an instruction that activates, deactivates or restricts a user input element (e.g. a button, a touch field or the like) on a user interface of the delivery device. The control commands may further update the controller, a possibly present electronic circuit or update a software program running in the delivery device.

The control commands may further comprise a drive command for a motor of the drug delivery device, for example, for automatically driving the drive member if a predefined criteria (for example a half full cartridge) is met.

For example, when reading that code information the controller can sets by means of an electric motor or a mechanical operation member a maximum settable dose, or/and a minimum settable dose, or a predefined dose interval (e. g. such that only fixed dose steps can be set) or the control commands defines that only a predefined dose can be dispensed. The control commands may further limit the dose and dispensing mechanism regarding a time table by defining, for example, a minimum time period between two consecutive administrations or enable the dispensing mechanism only at a predefined date or day time for dispensing a specific drug or a specific dose size may only be dispensed at a defined daytime or at defined period during a therapy.

Furthermore, a drug delivery system may comprise a first drug delivery device for dispensing a liquid drug, including
- an attachment structure for attaching a receptacle containing the liquid drug,
- a drive member for dispensing the drug from the receptacle,
- a code reader for reading code information from a code and
- a controller,
wherein the system may further comprise a second drug delivery device, wherein the first and the second drug delivery device are identical. The first and second drug delivery device may each comprise a receptacle provided with a first or a second code respectively, wherein the controller is adapted to set a delivery parameter (from a delivery instruction) and to enable or disable a function of the delivery device based on the first or second code information, wherein the delivery parameter and the enabled or disabled function differ between the first and second delivery device.

That means the delivery parameter and the available functions of the delivery device base on the code of the receptacle that is attached to the delivery device. Receptacles with different drugs and thus different codes may successively attached to the attachment structure of the delivery device. Therefore, the controller individually sets the delivery parameter and the device function for each drug based on the corresponding code information.

The delivery device is preferably an injection device comprising a cartridge unit including the receptacle and a drive unit including the drive member and the tag reader, wherein the cartridge unit is releasably attachable to the drive unit. Such devices are named as semi-disposable devices as the cartridge unit may be replaced and disposed of after an injection or if the cartridge is empty. Subsequently, a new cartridge unit may be attached to the reusable drive unit in order to prepare the device for a new injection.

The drive unit preferably comprises an electric motor for moving the drive member in a dispensing direction to dispense the liquid drug from the cartridge.

The drug delivery device may comprise a cartridge or reservoir with one component drug inside or the drug delivery device may comprise a cartridge with a two or more component drug. The latter has to be mixed by the user preferably prior an injection or an infusion.

Alternatively, the drug delivery device may be an infusion device such as a drug pump or a patch pump.

The invention relates further to a method for determining drug delivery instruction for a liquid drug with a drug delivery device, the method comprising the steps of
a. Reading, by a code reader, a machine-readable code of a receptacle comprising the liquid drug, the code comprising a first selection parameter associated with a first delivery instruction and a second selection parameter associated with a second delivery instruction;
b. Comparing, by a controller, an input value of the drug delivery device with the first and the second selection parameter;
c. Choosing, based on the comparison, the first or the second delivery instruction for delivery the liquid drug.

Preferably, the method is carried out by the controller of the drug delivery device. That means the method does not require any other (external) devices.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a top view of a semi-disposable injection pen according to the invention;
- Fig.2: depicts a perspective view of the pen of figure 1, wherein the disposable assembly and the reusable assembly are detached from each other;
- Fig.3: depicts an exploded view of the injection pen;
- Fig. 4: depicts a sectional view of the injection pen;
- Fig. 5: depicts a sectional view of a second embodiment of the pen with a distally arranged code reader.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

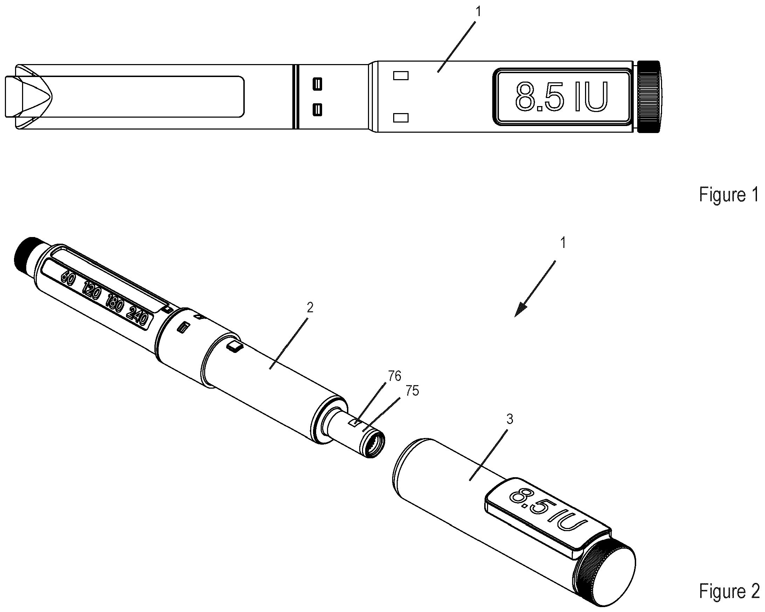

In the present description the term "distal" refers to the side where the needle is attached. This is on the left hand side in the figures 1 to 5. The term "proximal" refers to the opposite side and is on the right hand side in figures 1 to 5.

Figure 1 shows a drug delivery device according to the invention. In the embodiment shown in figure 1 the device is implemented as a semi-disposable injection pen 1. The pen 1 comprises a disposable assembly 2 and a reusable assembly 3, shown in Figure 2.

Figure 2 depicts the injection pen of figure 1 wherein the disposable assembly 2 is detached from the reusable assembly 3. The disposable assembly 2 is formed by a cartridge unit 2 (or dispensing unit) including a reservoir holder or cartridge holder and a reservoir in form of a cartridge containing liquid drug. The reusable assembly 3 includes a drive unit and a LCD unit with a display. The reusable assembly 3 is releasably attachable to the cartridge unit 2.

The cartridge unit 2 can be produced, pre-assembled and stored separately from the reusable assembly 3. That is, single parts of the cartridge unit 2 may be produced by a device manufacturer and delivered to the drug or medication manufacturer. The latter inserts the filled cartridge in the reservoir holder and pre-assembles the single components to the sub-assembled cartridge unit 2.

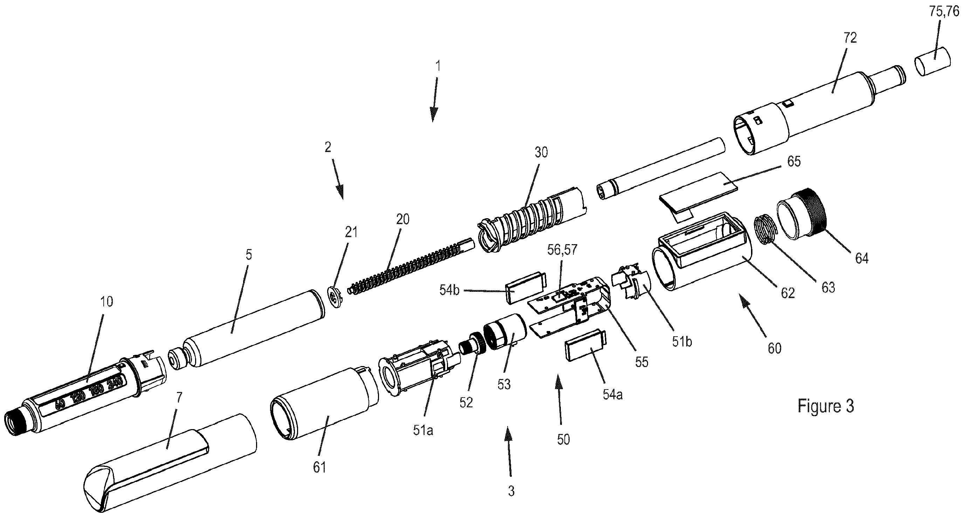

Figure 3 depicts a perspective and exploded view of the semi-disposable injection pen 1 with the single parts of the cartridge unit 2 and of the reusable assembly 3.

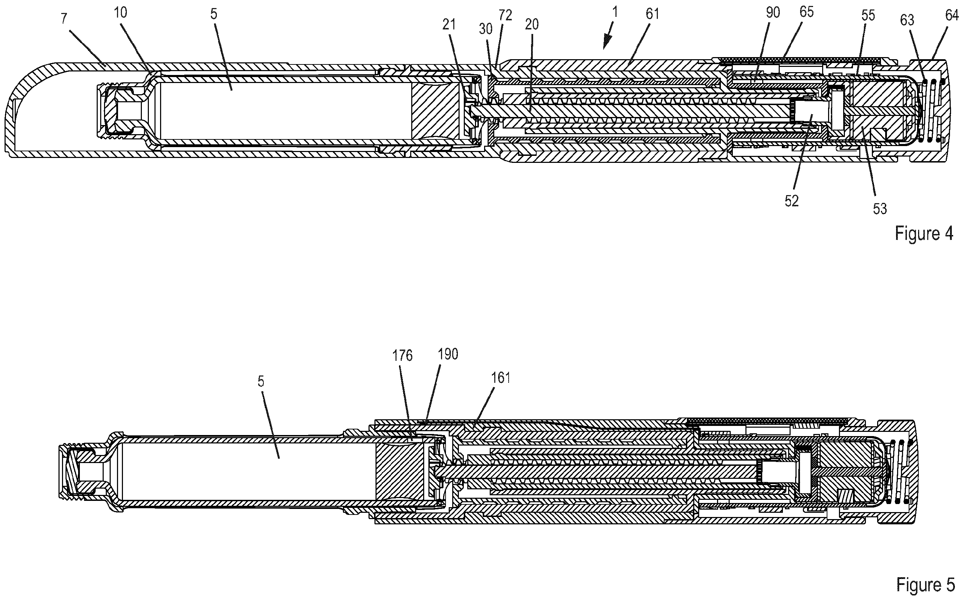

The reusable assembly 3 comprises an automatic drive unit 50 and the LCD unit 60. The automatic drive unit 50 comprises a support structure 51a, 51b, an electric motor 53 with a motor shaft, in particular a BLDC motor o stepper motor, for moving the plunger rod 20, a sleeve-shaped automatic drive member 52, a gear connecting the motor shaft and to an automatic drive member 52, a printed circuit board (PCB) 55 with a controller (not shown) for controlling the electric motor, an energy source in form of a rechargeable battery 54a, 54b, a tag reader 90 (see figure 4), a data storage module 57, a communication module 56 and an encoder (not shown) for sensing the movement of the motor shaft and thus the movement of the plunger rod 20. The LCD unit 60 includes a distal housing member 61 and a proximal housing member 62, a LCD 65, a spring 63 and a dose knob 64.

In an alternative embodiment the automatic drive unit may not comprise any display but only simple LEDs or no indication means at all. In this case the data are transmitted from the drive unit to a user device with a display for displaying that information.

The cartridge unit 2 includes an interface housing 72, the reservoir holder 10, the cartridge 5, a mechanics holder 30 and the plunger rod 20 with a flange 21 pivotally mounted on a distal end of the plunger rod 20. A pen cap 7 can be mounted on a needle assembly (not shown) attachable to a distal end of the reservoir holder 10.

The cartridge unit is further provided with a label 75 with a machine-readable tag 76 in form of a NFC tag, also depicted in figure 2. More precise the NFC tag 76 is mounted on a proximal end portion on an outer surface of the interface housing 72. The NFC tag 76 can be read by the tag reader 90 of the drive unit, shown in figure 4.

Figures 4 depicts a sectional view of the injection pen 1 wherein the cut runs along a longitudinal axis of the injection pen 1. If the cartridge unit 2 is attached to the reusable assembly 3 the tag reader 90 is positioned close to the NFC tag 76 of the cartridge unit 2. The position of the cartridge unit 2 in the attached state is given by a bayonet connection between the cartridge unit 2 and the reusable assembly 3.

In an alternative embodiment the cartridge unit code can be implemented as an OID code, a bar code, a QR code or an optical code. In the latter case the drive unit comprises an optical sensor configured to read the optical code.

Figure 5 shows a sectional view of an alternative embodiment of the injection pen 1. In contrast to the pen shown in figure 1 to 4 the NFC tag 176 is not arranged on an outside of the cartridge unit. Instead, the tag 176 is directly located on an outside of the cartridge 5. Accordingly, the tag reader 190 of the drive unit is located at a distal end of a distal housing member 161. The other structural and functional features of the injection pen are identical to the first embodiment. In particular, the following described features with respect to that tag information apply for all embodiments.

When a cartridge unit 2 is attached to the reusable assembly 3 the tag reader 90 of the drive unit can read that information from tag 76. The tag 76 comprises injection information including a UDI code, medication type and name, expiry date of the medication and volume of the cartridge 5.

The tag 76 comprises further drug delivery instructions in form of a plurality of drug-specific feed rates (delivery rates) for the plunger rod to dispense the drug from the cartridge 5. Each feed rate is associated with a selection parameter in form of a temperature range as the viscosity of the drug heavily depends on the temperature. Additionally, the tag comprises a plurality of drug-specific values of a holding time (dwell time). Each holding time value is associated to a temperature range and to a specific dose size.

A temperature sensor in the drive unit (not shown) measures a temperature of a surface of the cartridge holder. The detected temperature is representative of the drug temperature and the measured temperature is sufficient precise for the further processing. Subsequently, the controller compares the measured temperature (input value) with the temperature ranges given in that tag information (selection parameter). As a certain temperature range given in that tag information is associated with a certain feed rate and with a certain holding time the controller can choose the corresponding feed rate and the corresponding holding time. If, for example, the measured temperature is 22 °C the controller sets the feed rate to 0.5ml/min and the holding time to 4s as according to that read tag information these values are associated with a selection parameter of 20°C to 25°C.

Alternatively, the temperature sensor may be arranged in the cartridge unit. Consequently, the controller reads a temperature signal form the temperature sensor when the cartridge unit is attached to the reusable assembly.

Depending on the drug the tag comprises further information. Namely, a single dose usually set by the user can be selected or amended depending on a physiological body parameter such as the body weight of the user. Furthermore, the dose (maximum single dose) may depend on a specific therapy plan and can be set by the doctor. In this embodiment the controller receives the body weight via user interface or via communication module 56 form an external sender such as user device, a computer of a HCP or from a database on a cloud server. Consequently, the controller compares the received body weight value with value ranges read from the tag. As each value range given in that tag information is associated with a dose size the controller chooses the corresponding dose size or amends an already set dose. In other words, the dose size is chosen or an already set dose is amended depending on the user body weight.

In an alternative embodiment the physiological body parameter is a measured blood sugar value of the user. The controller choses the appropriate dose size provided by that code information and based on the actual blood sugar value. This ensures that an optimal dose size is set or recommended to the user.

In summary, the tag can include drug information with the following dependencies: feed rate depending on temperature, feed rate depending on dose size, feed rate depending on a specific therapy plan, feed rate depending on a body parameter, feed rate depending on a current plunger rod position (varying feed rate), feed rate depending on a user input (also as overwrite function), holding time depending on dose size, holding time depending on temperature, dose size depending on a specific therapy plan, dose size depending on a body parameter (e.g. body weight, blood sugar value and the like) or depending on a therapy parameter range, dose size (dose amount) depending on number of injection with the same drug (increasing/decreasing dose size) and dose size depending on spatial orientation of the injection pen (priming detection).

In a further embodiment a combination of the above function can be implemented. In such an embodiment that information of the NFC tag of the cartridge unit comprises fixed values for the feed rate and/or the holding time for the specific drug contained in the cartridge unit. However, the user can override some of these delivery parameters by a user input. That means, for example, the controller considers both the initial setting and a user input for setting the feed rate and/or holding time.

The dose size depending on a spatial orientation of the injection pen is advantageous to detect a priming operation. If the user holds the injection pen with the needle end upwards it is likely that the user intends to carry out a priming. Accordingly, instead of setting a dose the controller only moves the plunger rod a small distance to carry out a priming with the cartridge unit.

Additionally, tag information comprises threshold values. If a temperature value or a physiological body parameter (for example a user body temperature) exceeds a predefined threshold the controller prevents a dispensing movement of the motor. For example, if the measured drug temperature is above 35°C the controller does not drive the motor to dispense the drug as the measured temperate is in this case above the recommended injection temperature for the drug. In this case, warning information may be sent to the user to change and dispose the actual cartridge unit and replace it with a new one.

If a drug is only to be used for a specific body weight range that tag information indicates the body weight range accordingly. When receiving the body weight data the controller verifies whether the weight lies within the allowed weight range according to tag information and if the body weight is outside the scope of the drug the controller does not start the injection.

Furthermore, depending on the drug that tag information comprises a lock time that locks the motor of the automatic drive unit for a predefined time period after an injection in order to keep the required minimum time between to subsequent injections

**Connectivity** The above mention determination of the delivery parameter can be carried out autonomously. That means the injection pen does not require any data connection to an external device. In this case the therapy plan, if any, can be stored in the storage module 57 of the drive unit or the controller receives the dose via user input. In this embodiment the delivery parameter is completely determined based on tag information and on the measured input values.

Alternatively, the controller receives the dose to be injected via communication module 56 from an external source such as an external therapy plan on a cloud server or from a computer of a HCP wirelessly connected to the communication module 56. In the same manner after reading tag information from the cartridge unit the controller can verify read drug information internally (storage unit) or externally (cloud server). Such connectivity features are described in the European patent application EP20216386.1. Its disclosure is incorporated hereby by reference.

The pairing of the injection pen with another device or with a network (local network or internet) and in general the connection between the injection pen and another device or network can be initialized by the user or by the manufacturer at the manufacturer's site. In the latter case the data connection is not mandatory shown to the user ("hidden connectivity"). Accordingly, the subsequent tracking of delivery events, injection pen operations such as setting, correcting and administering a dose and replacing a cartridge or cartridge unit may be monitored and registered by the controller of the injection pen without user interaction ("hidden operation tracking").

Besides above mention information the code comprises additionally control commands for the injection pen. The controller receives the commands after reading the tag and depending on the drug the control commands restrict a maximum settable dose by controlling the motor 53. That means the controller blocks by means of the motor a rotation of the dose knob 64 if the maximum settable dose for a specific drug is reached. Furthermore, the user control elements (soft keys on the LCD 65) are adjusted according to the code control commands and depending on the drug. The control commands further comprise data and a command to update a software program running in the drive unit. If the controller reads the control commands of the tag the controller automatically starts the update function ensuring that the software program receives the latest data.

For example, for a drug A which is supposed to be administered in fixed doses the code of the cartridge unit comprises display commands for displaying soft keys on the LCD 65 with the fixed doses for the drug A. Besides that, based on the read commands the controller restricts the dose setting mechanism such that only the predefined fixed doses can be administered with drug A.

In a further an example for a drug B which is supposed to be administered based on a therapy plan the code of the cartridge unit 2 comprises commands for verifying the therapy plan. After reading code information the control command prompts the controller to send via communication module 56 that read code information to the therapy plan on the cloud server. Subsequently, the controller receives additional user specific information and displays an injection reminder, priming instructions and the dose size to be administered with drug B on the LCD 65 of the injection pen. The code further comprises a command for the controller to limit a maximum settable dose for the drug B.

Hence, the function, the delivery parameter but also available soft buttons on the LCD (user interface) are different for the drug A and the drug B.

The description above and the figures 1 to 5 are related to a semi-disposable injection pen. However, the code and the code reader according to the present invention can be implemented in any other drug delivery device such as disposable injection pen, a reusable injection pen, a semi-disposable autoinjector or a patch injector that is adapted to be attached on the user skin for a prolonged time period (e.g. several hours). In yet a further embodiment the code and code reader can be implemented in a drug infusion pump with a replaceable reservoir or in a patch pump.

With respect to a disposable and a reusable injection pen the code (NFC tag or RFID tag) is preferably arranged on an outer surface of the cartridge. The tag reader reads dispensing information from the code when a cartridge is inserted during manufacturing of the pen (disposable pen) or when the user inserts a cartridge or replaces a cartridge (reusable pen or semi-disposable pen). The injection pen does not mandatory need a drive unit or a motor. The controller can choose the delivery instruction and display the determined parameter on a display to the user. Subsequently, the users sets the displayed parameter and starts the drug delivery by manually moving the drive member in a dispensing direction.

With respect to a patch injector, a patch infusion pump, an infusion pump or any other infusion device the code is preferably arranged on an outside of the reservoir containing the drug. As described above a code reader in the device reads drug information if the reservoir is inserted into the device.

The injection pen shown in the figures 1 to 5 may comprise a cartridge containing a one component drug. However, the invention is not limited to such a drug type. In particular, drug information is advantageous for a device with a two or more component drug. In this case the two or more components of the drug have to be mixed before use. This can be done manually by the user or by an automatic plunger rod movement by the motor and controlled by the controller. The code arranged on the cartridge or on the cartridge unit as described above comprises in this embodiment additionally instruction for preparing the two component medication. After reading information from the tag the controller displays the mixing instructions on the display of the pen and/or on a user device. By means of accelerometer sensors or motion sensors in the injection pen the controller is adapted to monitor and confirm preliminary injection steps such as shaking the device or moving the plunger rod a short distance (forward or forwards and backwards) in order to mix the two components.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | Injection pen | 60 | Display unit |
| 2 | Cartridge unit (disposable assembly) | 61 | Distal housing member |
| 3 | reusable assembly | 62 | Proximal housing member |
| 5 | Cartridge | 63 | Spring |
| 7 | Pen cap | 64 | Dose knob |
| 10 | Cartridge holder | 65 | LCD |
| 20 | Plunger rod | 72 | interface housing |
| 21 | Flange | 75 | label with tag |
| 30 | Mechanics holder | 76 | tag |
| 50 | Automatic drive unit | 90 | tag reader |
| 51a,b | Support structure | 176 | tag |
| 51b | Support structure | 190 | tag reader |
| 52 | Automatic drive member | | |
| 53 | Electric motor | | |
| 54a,b | Battery | | |
| 55 | PCB | | |
| 56 | Communication module | | |
| 57 | Storage module | | |

## Claims

1. A drug delivery device (1) for dispensing a liquid drug, comprising
- a receptacle (2) adapted to contain the liquid drug and provided with a machine-readable code (76) including information;
- a drive member (20) for dispensing the drug from the receptacle (2);
- a code reader (90) for reading that information and
- a controller,
**characterized in that** code information comprises a first selection parameter value associated with a first drug delivery instruction and a second selection parameter value associated with a second drug delivery instruction and
wherein the controller is configured to compare an input value with the first and the second selection parameter value and to select the first or second drug delivery instruction based on the comparison.

2. The drug delivery device (1) according to claim 1, wherein the first and second selection parameter values are temperature values or temperature ranges and the input value is an instantaneous measured temperature value.

3. The drug delivery device (1) according to claim 1, wherein the first and second selection parameter values are dose values or dose value ranges and the input value is a dose value to be delivered by the drug delivery device.

4. The drug delivery device (1) according to claim 1, wherein the first and second selection parameter values are position values or position value ranges representing a position of the drive member (20) relative to a delivery device housing (61, 62) and the input value is a measured current position of the drive member (20).

5. The drug delivery device (1) according to any of claims 2 to 4, wherein the drug delivery instruction comprises a feed rate for the drive member (20).

6. The drug delivery device (1) according to claim 2 or claim 3, wherein the drug delivery instruction comprises a holding time to be indicated to the user.

7. The drug delivery device (1) according to claim 1, wherein the first and second selection parameter value are physiological body parameter or body parameter ranges and the input value is a physiological body parameter of the user.

8. The drug delivery device (1) according to claim 1, wherein the first and second selection parameter are user-specific therapy parameter or therapy parameter ranges and the input value is a physiological body parameter of the user.

9. The drug delivery device (1) according to claim 1, wherein the first and second selection parameter value are numbers or number ranges and the input value is a counted number of drug delivery events with the drug delivery device.

10. The drug delivery device (1) according to claim 1, wherein the first and second selection parameter value are values or value ranges representing a spatial orientation of the delivery device (1) and the input value is a measured spatial orientation of the delivery device (1).

11. The drug delivery device (1) according to any of claims 7 to 10, wherein injection instruction comprises a dose amount to be dispensed.

12. The drug delivery device (1) according to any of claims 1 to 11, wherein the drive member is prevented from a dispensing movement if the input value exceeds a predefined threshold.

13. The drug delivery device (1) according to any of claim 1 to 12, wherein the code further comprises instructions for use.

14. The drug delivery device (1) according to any of claim 1 to 13, wherein the code further comprises a control command for the drug delivery device (1).

15. Method for determining drug delivery instruction for a liquid drug with a drug delivery device (1), the method comprising the steps of
a. Reading, by a code reader (90), a machine-readable code (76) of a receptacle (2) comprising the liquid drug, the code (76) comprising code information including a first selection parameter value associated with a first drug delivery instruction and a second selection parameter value associated with a second drug delivery instruction;
b. Comparing, by a controller of the delivery device (1), an input value with the first and the second selection parameter value;
c. Choosing, based on the comparison, the first or the second drug delivery instruction for dispensing the liquid drug.
